# EUROPÄISCHE PATENTSCHRIFT

(11) **EP 1 451 242 B1**
(45) Veröffentlichungstag und Bekanntmachung des Hinweises auf die Patenterteilung: **20.02.2008**
(21) Anmeldenummer: 02787755.4
(22) Anmeldetag: 21.11.2002
(51) Int. Cl.: C08J 3/03

(54) **WASSER-IN-SILIKON-EMULSIONEN**
WATER-IN-SILICONE EMULSIONS
EMULSIONS DU TYPE EAU DANS SILICONE

(30) Priorität: 23.11.2001 DE 10157542
(43) Veröffentlichungstag der Anmeldung: 01.09.2004
(73) Patentinhaber: Beiersdorf AG, 20245 Hamburg (DE)
(72) Erfinder: BLECKMANN, Andreas, 22926 Ahrensburg (DE); FÜLLER, Silke, 22523 Hamburg (DE); KRÖPKE, Rainer, 22869 Schenefeld (DE); LONGREE, Inka, 22085 Hamburg (DE); SYSKOWSKI, Boris, 22303 Hamburg (DE)
(86) Internationale Anmeldenummer: PCT/EP2002/013053
(87) Internationale Veröffentlichungsnummer: WO 2003/044080

(56) Entgegenhaltungen:
- EP-A- 1 064 908
- US-A- 5 523 091
- US-A1- 2001 012 860

## Beschreibung

Die vorliegende Erfindung betrifft Wasser-in-Silikon-Emulsionen enthaltend Stärke und/oder Stärkederivate sowie deren Verwendung.

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öi-in-Wasser-Emutsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Neben den Emulsionen die aus Wasser und Ölen auf Kohlenstoff-Basis bestehen, gibt es auch solche, in denen Silikonöl als Lipidphase eingesetzt werden. Diese werden beispielsweise in der Kosmetik verwendet, um den Zubereitungen ein elegantes, seidiges Hautgefühl zu verleihen. Ferner können in diesen Emulsionen größere Mengen an Feuchthaltemitteln (Moisturizer), insbesondere Glycerin eingearbeitet werden, ohne dass die Zubereitung eine klebrige Sensorik bekommt.

Kosmetische und/oder dermatologische Wasser-in-Silikon-Emulsionen müssen jedoch üblicherweise mit Wachsen beziehungsweise Pigmenten als Stabilisatoren in relativ hoher Konzentration stabilisiert werden. Diese haben den Nachteil, dass sie sich nicht besonders gut auf der Haut verteilen lassen und nach der Anwendung einen klebrigen Rückstand auf der Haut zurücklassen. Bei höheren Mengenanteilen an Silikonölen in der Emulsion bleibt entsprechend viel Rückstand auf der Haut zurück und man erhält bei der Anwendung ein fettig-öliges Hautgefühl.

Es war daher die Aufgabe der vorliegenden Erfindung den Nachteilen des Standes der Technik abzuhelfen und langzeitstabile Wasser-in-Silikon-Emulsionen mit einem angenehmen Hautgefühl zu entwickeln.

Überraschend gelöst wird die Aufgabe durch Wasser-in-Silikon-Emulsionen enthaltend
a) eine wässrige Phase,
b) eine Lipidphase mit einem Silikonanteil von mindestens 50 Gewichts-%, bezogen auf die Lipidphase,
c) Emulgatoren,
d) Stärke und/oder Stärkederivate, gewählt aus der Gruppe Dimethylimidazolidon-Reisstärke, Aluminiumstärkeoctenylsuccinat und/oder Tapioka,
neben gegebenenfalls weiteren kosmetischen und/oder dermatologischen Wirk-, Hilfs- und Zusatzstoffen.

Diese Emulsionen ergeben bei ihrer kosmetischen und/oder dermatologischen Anwendung ein seidiges leichtes Hautgefühl in Kombination mit einem angenehm trockenen, pudrigen Rückstand auf der Haut. Darüber hinaus sind sie über 30 Monate bei Raumtemperatur lagerstabil. Diese positiven Eigenschaften bleiben sogar bei einem erhöhten Anteil an Feuchthaltemitteln in der Emulsion wie z.B. bei der Verwendung von über 10 Gewichts-% an Glycerin sowie bei Zusatz von bis zu 50 Gewichts-% unpolarer Lipide (z.B. Mineralöle, Vaseline, Dicaprylylether), bezogen auf das Gesamtgewicht der Lipidphase, erhalten.

Zwar kennt der Stand der Technik W/O-Emulsionen mit Silikomölen. So beschreiben die US 5,523,091, die EP 1 064 908 sowie die US 2001/0012860 derartige Zubereitungen, doch konnten diese Schriften nicht den Weg zur vorliegenden Erfindung weisen.

Die erfindungsgemäßen Wasser-in-Silikon-Emulsionen enthalten die erfindungsgemäße Stärke und/oder Stärkederivate in einer Menge von 0,1 bis 20 Gewichts-% und besonders bevorzugt 0,5 bis 10 Gewichts-%, bezogen auf das Gesamtgewicht der Emulsion.

Die erfindungsgemäß bevorzugten Stärken und/oder Stärkederivate sind Dimethylimidazolidon-Reisstärke [z.B. Rice NS (Dimethylimidazolidone Rice Starch) von Chemag], Aluminiumstärkeoctenylsuccinat [z.B. Dry Flo PC (Aluminium Starch Octenylsuccinate) von National Starch] und/oder Tapioka [= Manihotstärke, z.B.Tapioca Pure/ Tapioca Starch von National Starch].

Für die erfindungsgemäßen Wasser-in-Silikon-Emulsionen können alle bekannten für diesen Emulsionstyp verwendeten Emulgatoren eingesetzt werden. Erfindungsgemäß besonders bevorzugte Wasser-in-Silikon-Emulgatoren sind dabei Cetyldimethicon Copolyol und Laurylmethicon Copolyol [z.B. ABIL EM 90 (Goldschmidt), DC5200 Formulation Aid (Dow Corning)] sowie beliebige Mischungen aus beiden Emulgatoren.

Erfindungsgemäß ganz besonders vorteilhaft ist die Kombination aus Dimethylimidazolidon-Reisstärke [z.B. Rice NS (Dimethylimidazolidone Rice Starch) von Chemag] als Stärkederivat und Alkyldimethicon Copolyol [z.B. ABIL EM 90 (Goldschmidt), DC5200 Formulation Aid (Dow Corning)] als Emulgator, welche sowohl fließfähige Lotionen als auch Softcremes geringer Konsistenz und hoher Hautpflegeleistung, insbesondere bezüglich der Hautbefeuchtung sowie der Hautglättung ermöglichen.

Die Lipidphase der erfindungsgemäßen Wasser-in-Silikon-Emulsionen kann vorteilhaft gewählt werden aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Lipidphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner vorteilhaft, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, Cocoglyceride (z. B. Myritol® 331 von Henkel), C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Lipidphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*) und/oder Diethylhexylnaphthalat (*Hallbrite TQ von CP Hall oder Corapan®TQvon Haarmann & Reimer*).

Auch beliebige Abmischungen solcher Öl- und Wachskomponenten sind vorteilhaft im Sinne der vorliegenden Erfindung einzusetzen.

Die Lipidphase kann die polaren Ölkomponenten erfindungsgemäß in einer Konzentration von bis zu 40 Gewichts-% bezogen auf das Gesamtgewicht der Lipidphase enthalten.

Ferner kann die Lipidphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Die unpolaren Ölkomponenten können erfindungsgemäß in einer Konzentration von bis zu 50 Gewichts-% bezogen auf das Gesamtgewicht der Lipidphase in den erfindungsgemäßen Emulsionen enthalten sein.

Die erfindungsgemäße Lipidphase enthält ferner Silikonöle. Sie kann einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 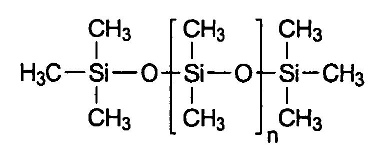 werden auch als Polydimethylsiloxan bzw. Dimethicon (INCI) bezeichnet. Dimethicone gibt es in verschiedenen Kettenlängen bzw. mit verschiedenen Molekulargewichten.

Vorteilhafte Polyorganosiloxane im Sinne der vorliegenden Erfindung sind beispielsweise Dimethylpolysiloxan [Poly(dimethylsiloxan)], welche beispielsweise unter den Handelsbezeichnungen Abil 10 bis 10 000 bei Goldschmidt erhältlich sind. Ferner vorteilhaft sind Phenylmethylpolysiloxan (INCI: Phenyl Dimethicon, Phenyl Trimethicon), cyclische Silikone (Octamethylcyclotetrasiloxan bzw. Decamethylcyclopentasiloxan), welche nach INCI auch als Cyclomethicon bezeichnet werden, aminomodifizierte Silikone (INCI: Amodimethicone) und Silikonwachse, z. B. Polysiloxan-Polyalkylen-Copolymere (INCI: Stearyl Dimethicon und Cetyl Dimethicon) und Dialkoxydimethylpolysiloxane (Stearoxy Dimethicon und Behenoxy Stearyl Dimethicon), welche als verschiedene Abil-Wax-Typen bei Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Erfindungsgemäß besonders bevorzugte Silikone sind Dimethicon und Cyclomethicon.

Der Silikonölanteil der Lipidphase kann erfindungsgemäß 50 bis 100 Gewichts-% und besonders bevorzugt von 75 bis 95 Gewichts-%, jeweils bezogen auf das Gesamtgewicht der Lipidphase betragen.

Die wässrige Phase der erfindungsgemäßen Emulsionen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, Polymere, Schaumstabilisatoren, Elektrolyte, Dihydoxyaceton sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Ferner vorteilhaft sind Copolymere aus C₁₀₋₃₀-Alkylacrylaten und einem oder mehreren Monomeren der Acrylsäure, der Methacrylsäure oder deren Ester.

Vorteilhaft sind Verbindungen, die die INCI-Bezeichnung "Acrylates/C₁₀₋₃₀ Alkyl Acrylate Crosspolymer" tragen. Insbesondere vorteilhaft sind die unter den Handelsbezeichnungen Pemulen TR1 und Pemulen TR2 bei der B. F. Goodrich Company erhältlichen.

Vorteilhaft sind Verbindungen, die die INCI-Bezeichnung Ammoniumacryloyldimethyltaurate/Vinylpyrrolidoncopolymere tragen.

Erfindungsgemäß vorteilhaft weisen das oder die Ammoniumacryloyldimethyltaurate/Vinylpyrrolidoncopolymere die Summenformel [C₇H₁₆N₂SO₄]ₙ [C₆H₉NO]ₘ auf, einer statistischen Struktur wie folgt entsprechend

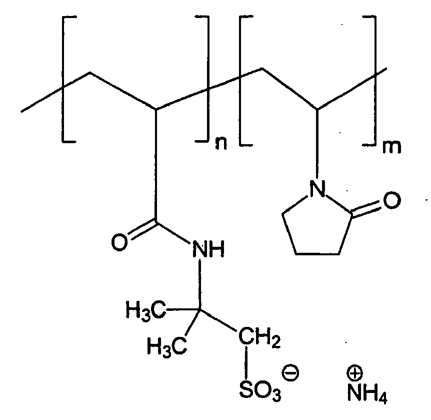

Bevorzugte Spezies im Sinne der vorliegenden Erfindung sind in den Chemical Abstracts unter den Registraturnummern 58374-69-9, 13162-05-5 und 88-12-0 abgelegt und erhältlich unter der Handelsbezeichnung Aristoflex® AVC der Gesellschaft Clariant GmbH.

Vorteilhaft sind ferner Copolymere/Crosspolymere umfassend Acryloyldimethyl Taurate, wie beispielsweise Simugel ® EG oder Simugel ® EG von der Gesellschaft Seppic S.A.

Weitere erfindungsgemäß vorteilhaft zu verwendende Verdickungsmittel sind auch in Wasser lösliche oder dispergierbare anionische Polyurethane. Vorteilhaft im Sinne der vorliegenden Erfindung sind z. B. Polyurethan-1 und/oder Polyurethan-4.

Besonders vorteilhafte Polyurethane im Sinne der vorliegenden Erfindung sind die unter der Handelsbezeichnung Avalure^{™} UR bei der B. F. Goodrich Company erhältlichen Typen, wie beispielsweise Avalure^{™} UR 445, Avalure^{™} UR 450 und dergleichen. Ferner vorteilhaft im Sinne der vorliegenden Erfindung ist auch das unter der Handelsbezeichnung Luviset Pur bei der BASF erhältliche Polyurethan.

Auch Feuchthaltemittel (Moisturizer) können bevorzugt verwendet werden. Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Es ist erfindungsgemäß vorteilhaft, wenn die Wasser-in-Silikon-Emulsion Feuchthaltemittel in einer Konzentration von 5 bis 25 Gewichts-% und besonders bevorzugt in einer Konzentration von 7,5 bis 15 Gewichts-%, jeweils bezogen auf das Gesamtgewicht der Emulsion enthält.

Das Verhältnis von wässriger Phase zu Lipidphase beträgt in den erfindungsgemäßen Wasser-in-Silikon-Emulsionen vorteilhafterweise 65:35 bis 80:20, wobei das Verhältnis von 70:30 bis 75:25 (wässrige Phase zu Lipidphase) besonders bevorzugt ist.

Die Wasser-in-Silikon-Emulsionen gemäß der Erfindung können kosmetische Hilfsstoffe enthalten, wie sie üblicherweise in solchen Zubereitungen verwendet werden, z. B. Konservierungsmittel, Konservierungshelfer, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate. Ferner können sie organische und/oder anorganische öllösliche, wasserlösliche und/oder pigmentäre UV-Lichtschutzfilter enthalten.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant ^{™} von der Fa. Lonza erhältlich ist), lodopropylbutylcarbamate (z. B. die unter den Handelsbezeichnungen Glydant-2000, Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfasst das Konservierungssystem erfindungsgemäß ferner vorteilhaft auch Konservierungshelfer, wie beispielsweise Octoxyglycerin, Glycine Soja etc.

Besonders vorteilhafte Zubereitungen werden ferner erhalten, wenn als Zusatz- oder Wirkstoffe Antioxidantien eingesetzt werden. Erfindungsgemäß enthalten die Emulsionen vorteilhaft ein oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder β-Alanin.

Erfindungsgemäße Rezepturen, welche z. B. bekannte Antifaltenwirkstoffe wie Flavonglycoside (insbesondere *α*-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die erfindungsgemäßen Wasser-in Silikon-Emulsionen können wie übliche kosmetische und/oder dermatologische Zubereitungen zusammengesetzt sein und dem kosmetischen oder dermatologischen Lichtschutz, ferner zur Behandlung, Pflege und Reinigung der Haut und/oder der Haare und als Schminkprodukt in der dekorativen Kosmetik dienen.

Entsprechend ihrem Aufbau können Wasser-in-Silikon-Emulsionen im Sinne der vorliegenden Erfindung, beispielsweise verwendet werden als Hautschutzcrème, Reinigungsmilch, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Zur Anwendung werden die Wasser-in Silikon-Emulsionen in der für Kosmetika üblichen Weise auf die Haut und/oder die Haare in ausreichender Menge aufgebracht.

Erfindungsgemäß ist auch die Verwendung von Stärke und/oder Stärkederivaten zur Stabilisierung von Wasser-in-Silikon-Emulsionen.

Erfindungsgemäß ist weiterhin die Verwendung von Wasser-in-Silikon-Emulsionen als kosmetische und/oder dermatologische Zubereitung.

Ferner ist die Verwendung von Wasser-in-Silikon-Emulsionen als kosmetische und/oder dermatologische Creme oder Lotion erfindungsgemäß.

Nicht zuletzt ist die Verwendung von Wasser-in-Silikon-Emulsionen als Lotion oder Creme zur Hautpflege, Hautbefeuchtung und/oder Hautglättung erfindungsgemäß.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | **1** | **2** | **3** | **4** | **5** |
|---|---|---|---|---|---|
| Laurylmethicon Copolyol | 3 | 2 | | | 0,5 |
| Cetyl Dimethicon Copolyol | | | 3 | 2 | 1,5 |
| | | | | | |
| Cera Microcrystallina | | 1,5 | | 1,5 | 2,5 |
| Petrolatum | | | | | 1,5 |
| Cyclomethicon | 25 | 18 | 25 | 18 | 20 |
| Dimethicon | 2,5 | 2 | 2,5 | 2 | 1 |
| Phenyltrimethicon | 1 | 0,5 | 2,5 | 1 | |
| Dimethiconol | 0,5 | 1 | 0,5 | 1 | |
| | | | | | |
| Phenoxyethanol | 0,5 | 0,5 | 0,5 | 0,5 | |
| Parabene | | 0,2 | | 0,2 | 0,1 |
| Formaldehydabspalter | 0,3 | | 0,3 | | 0,1 |
| lodopropynyl Butylcarbamat | | 0,1 | | 0,1 | 0,1 |
| | | | | | |
| Magnesiumsulfat | | 0,7 | 1,2 | | 0,5 |
| Natriumchlorid | 1,5 | | | 2 | 0,8 |
| | | | | | |
| Propylen Glykol | 3 | 5 | | 3 | |
| Butylen Glykol | 4 | 6 | | 5 | |
| Glycerin | 6 | 8 | 10 | 12 | 20 |
| Alkohol Denat. | 3 | | 5 | | |
| | | | | | |
| Dimethylimidazolidon-Reisstärke | 1 | | 1 | | 0,5 |
| Aluminiumstärkeoctenylsuccinat | | 1 | | 2 | 0,5 |
| Tapiokastärke | | 0,5 | | | 0,5 |
| | | | | | |
| Parfüm | q.s. | q.s. | q.s. | q.s. | q.s. |
| Wasser | ad 100 | ad 100 | ad 100 | ad 100 | ad 100 |

## Patentansprüche

1. Wasser-in Silikon-Emulsion enthaltend
a) eine wässrige Phase,
b) eine Lipidphase mit einem Silikonanteil von mindestens 50 Gewichts-%, bezogen auf die Lipidphase,
c) Emulgatoren,
d) Stärke und/oder Stärkederivate, gewählt aus der Gruppe Dimethylimidazolidon-Reisstärke, Aluminiumstärkeoctenylsuccinat und/oder Tapioka,
neben gegebenenfalls weiteren kosmetischen und/oder dermatologischen Wirk-, Hilfs- und Zusatzstoffen.

2. Wasser-in-Silikon-Emulsion nach Anspruch 1, **dadurch gekennzeichnet, dass** Stärke und/oder Stärkederivate in einer Menge von 0,1 bis 20 Gewichts-% bezogen auf das Gesamtgewicht der Emulsion eingesetzt werden.

3. Wasser-in-Silikon-Emulsion nach einem der Ansprüche 1 bis 2, **dadurch gekennzeichnet, dass** als Emulgatoren Cetyldimethicon Copolyol und/oder Laurylmethicon Copolyol eingesetzt werden.

4. Wasser-in-Silikon-Emulsion nach einem der Ansprüche 1 bis 3, **dadurch gekennzeichnet, dass** sie eine Kombination aus Dimethylimidazolidon-Reisstärke als Stärkederivat und Alkyldimethicone Copolyol als Emulgator enthalten.

5. Wasser-in-Silikon-Emulsion nach einem der Ansprüche 1 bis 4, **dadurch gekennzeichnet, dass** als Silikone Dimethicon und/oder Cyclomethicon eingesetzt werden.

6. Wasser-in-Silikon-Emulsion nach einem der Ansprüche 1 bis 5, **dadurch** gekenn-zeichnet, dass die Emulsion Feuchthaltemittel in einer Konzentration von 5 bis 25 Gewichts-% und besonders bevorzugt in einer Konzentration von 7,5 bis 15 Gewichts-% bezogen auf das Gesamtgewicht der Emulsion enthält.

7. Wasser-in-Silikon-Emulsionen nach einem der Ansprüche 1 bis 6, **dadurch gekennzeichnet, dass** der Silikonölanteil der Lipidphase 50 bis 100 Gewichts-% und besonders bevorzugt von 75 bis 95 Gewichts-%, jeweils bezogen auf das Gesamtgewicht der Lipidphase beträgt.

8. Wasser-in-Öl-Emulsion nach einem der Ansprüche 1 bis 7, **dadurch gekennzeichnet, dass** das Verhältnis von wässriger Phase zu Lipidphase 65:35 bis 80:20 und besonders bevorzugt 70:30 bis 75:25 (wässrige Phase zu Lipidphase) beträgt.

9. Verwendung von Särke und/oder Stärkederivaten nach einem der vorhergehenden Ansprüche zur Stabilisierung von Wasser-in-Silikon-Emulsionen.

10. Verwendung von Wasser-in-Silikon Emulsionen nach einem der vorhergehenden Ansprüche als kosmetische und/oder dermatologische Zubereitung.

11. Verwendung von Wasser-in-Silikon-Emulsionen nach einem der vorhergehenden Ansprüche als kosmetische und/oder dermatologische Creme oder Lotion.

12. Verwendung von Wasser-in-Silikon-Emulsionen nach einem der vorhergehenden Ansprüche als Lotion oder Creme zur Hautpflege, Hautbefeuchtung und/oder Hautglättung.

## Claims

1. A water-in-silicone emulsion comprising
a) an aqueous phase,
b) a lipid phase having a silicone content of at least 50% by weight, based on the lipid phase,
c) emulsifiers,
d) starch and/or starch derivatives selected from the group consisting of dimethylimidazolidone-rice starch, aluminum starch octenylsuccinate and/or tapioca,
beside, if appropriate, further cosmetic and/or dermatological active ingredients, excipients and additives.

2. The water-in-silicone emulsion as claimed in claim 1, **characterized in that** starch and/or starch derivatives are employed in an amount of from 0.1 to 20% by weight based on the total weight of the emulsion.

3. The water-in-silicone emulsion as claimed in one of claims 1 to 2, **characterized in that** the emulsifiers employed are cetyldimethicone copolyol and/or laurylmethicone copolyol.

4. The water-in-silicone emulsion as claimed in one of claims 1 to 3, **characterized in that** it contains a combination of dimethylimidazolidone rice starch as the starch derivative and alkyldimethicone copolyol as the emulsifier.

5. The water-in-silicone emulsion as claimed in one of claims 1 to 4, **characterized in that** the silicones employed are dimethicone and/or cyclomethicone.

6. The water-in-silicone emulsion as claimed in one of claims 1 to 5, **characterized in that** the emulsion contains humectants in a concentration of 5 to 25% by weight and particularly preferably in a concentration of 7.5 to 15% by weight based on the total weight of the emulsion.

7. The water-in-silicone emulsion as claimed in one of claims 1 to 6, **characterized in that** the silicone content of the lipid phase is 50 to 100% by weight and particularly preferably 75 to 95% by weight, in each case based on the total weight of the lipid phase.

8. The water-in-silicone emulsion as claimed in one of claims 1 to 7, **characterized in that** the ratio of aqueous phase to lipid phase is 65:35 to 80:20 and particularly preferably 70:30 to 75:25 (aqueous phase to lipid phase).

9. The use of starch and/or starch derivatives as claimed in one of the preceding claims for the stabilization of water-in-silicone emulsions.

10. The use of water-in-silicone emulsions as claimed in one of the preceding claims as a cosmetic and/or dermatological preparation.

11. The use of water-in-silicone emulsions as claimed in one of the preceding claims as a cosmetic and/or dermatological cream or lotion.

12. The use of water-in-silicone emulsions as claimed in one of the preceding claims as a lotion or cream for skincare, moisturizing the skin and/or smoothing the skin.

## Revendications

1. Emulsion eau-dans-silicone contenant
a) une phase aqueuse
b) une phase lipidique contenant une proportion de silicone d'au moins 50% en poids par rapport à la phase lipidique,
c) des émulsifiants,
d) de l'amidon et/ou des dérivés d'amidon, choisis dans le groupe formé par les amidons de riz de type diméthylimidazolidone, l'octénylsuccinate d'amidon d'aluminium et/ou du tapioca,
outre le cas échéant d'autres substances actives, adjuvants et additifs cosmétiques et/ou dermatologiques.

2. Emulsion eau-dans-silicone selon la revendication 1, **caractérisée en ce que** l'amidon et/ou les dérivés d'amidon sont utilisés en une quantité de 0,1 à 20% en poids par rapport au poids total de l'émulsion.

3. Emulsion eau-dans-silicone selon l'une quelconque des revendications 1 à 2, **caractérisée en ce qu'**on utilise comme émulsifiants du Cetyldimethicon Copolyol et/ou du Laurylmethicon Copolyol.

4. Emulsion eau-dans-silicone selon l'une quelconque des revendications 1 à 3, **caractérisée en ce qu'**elle contient une combinaison d'amidons de riz de type diméthylimidazolidone comme dérivé d'amidon et de l'Alkyldimethicon Copolyol comme émulsifiant.

5. Emulsion eau-dans-silicone selon l'une quelconque des revendications 1 à 4, **caractérisée en ce qu'**on utilise, comme silicones du Dimethicon et/ou du Cyclomethicon.

6. Emulsion eau-dans-silicone selon l'une quelconque des revendications 1 à 5, **caractérisée en ce que** l'émulsion contient des agents d'hydratation en une concentration de 5 à 25% en poids et de manière particulièrement préférée en une concentration de 7,5 à 15% en poids par rapport au poids total de l'émulsion.

7. Emulsions eau-dans-silicone selon l'une quelconque des revendications 1 à 6, **caractérisées en ce que** la proportion d'huile de silicone de la phase lipidique représente 50 à 100% en poids et de manière particulièrement préférée 75 à 95% en poids par rapport au poids total de la phase lipidique.

8. Emulsion eau-dans-huile selon l'une quelconque des revendications 1 à 7, **caractérisée en ce que** le rapport de la phase aqueuse à la phase lipidique est de 65:35 à 80:20 et de manière particulièrement préférée de 70:30 à 75:25 (phase aqueuse à phase lipidique).

9. Utilisation d'amidon et/ou de dérivés d'amidon selon l'une quelconque des revendications précédentes pour la stabilisation d'émulsions eau-dans-silicone.

10. Utilisation d'émulsions eau-dans-silicone selon l'une quelconque des revendications précédentes comme préparation cosmétique et/ou dermatologique.

11. Utilisation d'émulsions eau-dans-silicone selon l'une quelconque des revendications précédentes comme crème ou lotion cosmétique et/ou dermatologique.

12. Utilisation d'émulsions eau-dans-silicone selon l'une quelconque des revendications précédentes comme lotion ou crème pour le soin de la peau, l'hydratation de la peau et/ou le lissage de la peau.
